# EUROPÄISCHE PATENTSCHRIFT

(11) **EP 2 814 452 B1**
(45) Veröffentlichungstag und Bekanntmachung des Hinweises auf die Patenterteilung: **04.01.2017**
(21) Anmeldenummer: 12781360.8
(22) Anmeldetag: 09.11.2012
(51) Int. Cl.: A61K 8/25, A61Q 5/06, A61K 8/02, A61K 8/11, A61K 8/894, A61K 8/81, A61K 8/86, A61K 8/891, A61K 8/92

(54) **PULVERFÖRMIGE KOSMETIKA**
POWDERY COSMETICS
PRODUITS COSMÉTIQUES PULVÉRULENTS

(30) Priorität: 16.12.2011 DE 102011088815
(43) Veröffentlichungstag der Anmeldung: 24.12.2014
(73) Patentinhaber: Henkel AG & Co. KGaA, 40589 Düsseldorf (DE)
(72) Erfinder: KNAPPE, Thorsten, 22869 Schenefeld (DE); HENSCHEL, Anna, 21423 Winsen (Luhe) (DE)
(86) Internationale Anmeldenummer: PCT/EP2012/072254
(87) Internationale Veröffentlichungsnummer: WO 2013/087315

(56) Entgegenhaltungen:
- WO-A1-2011/076518
- DE-A1-102007 058 921
- DE-A1-102008 057 261
- US-B1- 6 290 939
- DATABASE GNPD [Online] MINTEL; Dezember 2010 (2010-12), "Fast-Acting Bleaching Powder", XP002730561, Database accession no. 1443185
- DATABASE GNPD [Online] MINTEL; Oktober 2007 (2007-10), "Loose Powder", XP002730562, Database accession no. 790512

## Beschreibung

Die vorliegende Erfindung betrifft das technische Gebiet des Einsatzes pulverförmiger Zusammensetzungen zur kosmetischen Behandlung keratinischer Fasern, insbesondere menschlicher Haare.

Pulverförmige Kosmetika sind dem Fachmann lange bekannt. Diese werden etwa im Bereich der Hautbehandlung bereits seit langem eingesetzt. Typische Beispiele sind etwa Make-up Puder oder Lidschatten. Weiterhin sind für den Einsatz der Haarbehandlung tensidfreie aber lösemittelhaltige Trockenshampoos, die mittels Aerosolvorrichtung auf die Fasern gesprüht werden lange im Einsatz. Die im Trockenshampoo enthaltenen Stärkepartikel nehmen nach Verdunstung des Lösemittels Sebum und Schmutz auf und werden nach einer Einwirkzeit ausgebürstet. Ferner kennt der Fachmann Pulverhaarfarben, die jedoch nicht in Pulverform auf die Fasern aufgetragen werden, sondern zuvor mit separat zugefügtem Wasser unter Ausbildung einer Färbecreme verrührt werden. Die resultierende Creme findet sodann Anwendung auf dem Haar.

Stylingmittel zur Verformung keratinischer Fasern sind im Allgemeinen lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle. Temporäre Formgebungen, die einen guten Halt ergeben sollen, ohne das gesunde Aussehen der Haare, wie zum Beispiel deren Glanz, zu beeinträchtigen, können beispielsweise durch Haarsprays, Haarwachse, Haargele, Fönwellen etc. erzielt werden.

Entsprechende Mittel zur temporären Formgebung enthalten als formgebende Komponente üblicherweise synthetische Polymere. Zubereitungen, die ein Polymer enthalten, können mittels Treibgasen oder durch einen Pumpmechanismus auf das Haar aufgebracht werden. Haargele und Haarwachse werden hingegen in der Regel nicht direkt auf das Haar appliziert, sondern mittels eines Kamms oder der Hände im Haar verteilt.

Bekannte Formen temporärer Stylingmittel lassen sich oftmals nicht mit zufriedenstellender Genauigkeit dosieren. So lassen sich etwa Haargele, Haarcremes und Haarwachse nur noch schwierig verteilen, sobald sie einmal auf das Haar aufgebracht sind. Sobald der Kamm oder die Hände, auf die das Stylingmittel aufgebracht wurde, mit den ersten Haarpartien in Kontakt kommen, werden vergleichsweise große Mengen an Stylingmittel an das Haar abgegeben. In Haarpartien, die erst später mit dem Kamm oder den Händen erreicht werden, wird hingegen vergleichsweise wenig Stylingmittel eingearbeitet. Dies hat zur Folge, dass der Anwender entweder von Anfang an eine große Menge Stylingmittel aufbringen muss, so dass auch die zuletzt erreichten Haarpartien ausreichend Stylingmittel erhalten, oder gezwungen ist, das Stylingmittel in mehreren Schritten aufzubringen, wobei jeweils andere Haarpartien behandelt werden. Haarsprays lassen sich gleichmäßiger auf das Haar verteilen. Da der Verwender aber keine Möglichkeit hat, die Gesamtmenge an aufgebrachtem Stylingmittel visuell zu erfassen, besteht die Gefahr, dass mehr Stylingmittel auf das Haar aufgebracht wird, als eigentlich erforderlich wäre.

Die Druckschrift WO 2007/051511 A1 offenbart die Verwendung einer pulverförmigen Zusammensetzung, enthaltend 50 bis 95 Gew.-% eines wässrigen Lösungsmittels, hydrophobiertes Siliziumdioxidpulver und mindestens im wässrigen Lösemittel befindliches filmbildendes und/oder festigendes Polymer zur temporären Verformung keratinischer Fasern.

Die Druckschrift WO 2010/054980 A1 offenbart die Verwendung einer pulverförmigen Zusammensetzung mit Kern-Hülle-Teilchen zur temporären Verformung keratinischer Fasern, wobei die Hülle die Partikel mindestens eines hydrophobierten Metalloxidpulvers enthält und der Kern eine flüssige, wässrige Phase umfasst. Diese pulverförmigen Kern-Hülle-Teilchen umfassen mindestens ein filmbildendes und/oder festigendes Polymer in Form von Partikeln.

In der Druckschrift WO-A1-2011/076518 werden pulverförmige Zusammensetzungen beschrieben, die sich in eine Flüssigkeit umwandeln lassen und die einen auf einem Träger sorbierten Wirkstoff umfassen.

Insbesondere auf dem Gebiet der Haarkosmetik zeigen die aus dem Stand der Technik bekannten pulverförmigen Zusammensetzungen ein beschränktes Anwendungsprofil.

Aufgabe der vorliegenden Erfindung war es daher, eine pulverförmiges Zusammensetzung zur kosmetischen Behandlung keratinischer Fasern zur Verfügung zu stellen, die im Rahmen aller Ausführungsformen
- genau und einfach dosiert werden kann,
- sich ohne zu verklumpen gut auf der keratinhaltigen Faser verteilen lässt,
- als leave-on Kosmetikum (i.e. Kosmetikum wird nach Applikation nicht vom Substrat gespült/gewaschen) auf der Faser einen sichtbaren Matteffekt vermeidet (aber dennoch durch Zusatz geeigneter Inhaltsstoffe als mattierende Zusammensetzung eingesetzt werden könnte),
- als rinse-off Kosmetikum (i.e. Kosmetikum wird nach Applikation vom Substrat gespült/gewaschen) sich gut von der Faser abspülen/abwaschen lässt,
- eine verbesserte Lagerstabilität hat, und
- kosmetische Wirkstoffe schnell und zuverlässig an das Substrat abgibt.

Folgende konkretere Aufgaben im Rahmen der o.g. Grundprobleme existieren für die Erfindung im Zusammenhang folgender möglicher Ausführungsformen:
In der Ausführungsform als pulverförmige Zusammensetzung mit Eignung zur Verformung keratinischer Fasern, insbesondere menschlicher Haare, sollten die pulverförmigen Zusammensetzungen auch mit den im Bereich des Haarstylings üblichen wachsförmigen Estern kompatibel und stabil sein. Bei Anwendung der wachsesterhaltigen pulverförmigen Zusammensetzung sollte sich das Pulver in eine flüssige bis pastöse Form/Konsistenz transformieren lassen. Diese pulverförmigen Zusammensetzungen werden überwiegend als leave-on Kosmetikum eingesetzt.

In der Ausführungsform als pulverförmige Zusammensetzung mit Eignung zur Verformung keratinischer Fasern, insbesondere menschlicher Haare, sollten die pulverförmigen Zusammensetzungen keine feinen Stäube von in der Zusammensetzung als Feststoff enthaltenden, festigenden Polymeren bilden. Darüber hinaus soll sich das festigende Polymer gut aus der pulverförmigen Zusammensetzung freisetzen und sich gleichmäßig auf dem Substrat verteilen lassen Diese pulverförmigen Zusammensetzungen werden überwiegend als leave-on Kosmetikum eingesetzt.

Es wurde gefunden, dass die Lehre des Standes der Technik durch eine nachfolgend beschriebene pulverförmige Zusammensetzung entsprechend verbessert wird.

Ein erster Gegenstand der vorliegenden Erfindung ist Verwendung einer pulverförmigen Zusammensetzung, enthaltend
- mindestens eine partikelförmige, amorphe Fällungskieselsäure, mit der Maßgabe, dass in besagter Fällungskieselsäure mindestens ein kosmetischer Wirkstoff sorbiert vorliegt, der ausgewählt wird unter wachsförmigen Estern;
- Kern-Hülle-Teilchen, deren Hülle Partikel mindestens eines hydrophobierten Metalloxidpulvers enthält und deren Kern eine flüssige, wässrige Phase umfasst,
zur temporären Verformung keratinhaltiger Fasern.

Mittel zur temporären Verformung keratinischer Fasern werden in den deutschen Patentanmeldungen DE 10 2008 057261 und DE 10 2007 058921 beschrieben.

Das US-amerikanische Patent US 6 290 939 B1 beschreibt den Einsatz von Fällungskieselsäure (hydrated silica) in kosmetischen Mitteln.

Partikel sind im Sinne der Erfindung als Korn vorliegende Teilchen (vgl. DIN 66160: 1992-09) von Festkörpern.

Pulverförmig im Sinne der Erfindung sind Zusammensetzungen, deren Teilchen unter eigenem Gewicht frei rieselfähig sind (vgl. DIN EN ISO 6186: 1998-08).
Ein amorpher Zustand eines Festkörpers liegt vor, wenn dessen atomare Bausteine überwiegend nicht in Kristallgittern angeordnet sind. Anders als bei einer kristallinen Substanz, bei der neben einer Nahordnung zwischen den Bausteinen (d. h. konstante Abstände und Winkel zu den nächsten Nachbaratomen) auch eine Fernordnung (regelmäßige Wiederholung eines Motivs in einem Kristallgitter) existiert, liegt im amorphen Zustand lediglich eine mehr oder minder ausgeprägte Nahordnung vor.

Sorption ist die Sammelbezeichnung für alle Vorgänge (u.a. Absorption, Adsorption), bei denen ein Stoff durch einen anderen mit ihm in Berührung stehenden Stoff selektiv aufgenommen wird. Das partikelförmige, amorphe Metalloxid ist das Sorbens, der kosmetische Wirkstoff das Sorbat.

Wenn Mischungen der besagten kosmetischen Wirkstoffe vorliegen, können mehrere verschiedene Wirkstoffe gemeinsam auf demselben Partikel des partikelförmigen amorphen Metalloxids sorbiert vorliegen (gemeinsame Sorption).

Darüber hinaus können verschiedene Wirkstoffe einzeln auf Partikel eines amorphen Metalloxids sorbiert werden. Auf den Partikeln liegt somit nur ein Wirkstoff sorbiert vorliegt. Diese Partikel werden dann gemischt (getrennte Sorption). Es entsteht dabei eine pulverförmige Zusammensetzung mit einer auf partikelförmigen amorphen Metalloxiden sorbierten Wirkstoffmischung, wobei auf jedem Partikel dieser Mischung nur einer der kosmetischen Wirkstoffe sorbiert vorliegt.

Im Rahmen dieser Anmeldung ist das partikelförmige, amorphe Metalloxid eine Fällungskieselsäure. Als Fällungskieselsäure eignet sich wiederum bevorzugt eine Fällungskieselsäure mit einem Anteil von 0,1 bis 1,0 Gew.-% Na₂O bezogen auf ihr Gewicht.

Ferner zeigte es sich im Rahmen einer bevorzugten Ausführungsform, dass die erfindungsgemäße Aufgabe besonders gut gelöst wird, wenn das partikelförmige, amorphe Metalloxid eine DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid besitzt. Die DBP-Zahl wird stets in g DBP (Dibutylphthalat) pro 100 g Metalloxid angegeben und nach der Vorschrift der ASTM D 2414 basierend auf der getrockneten Substanz bestimmt.

Die Freisetzung des sorbierten kosmetischen Wirkstoff erfolgt besonders gut, wenn das partikelförmige, amorphe Metalloxid der erfindungsgemäßen pulverförmigen Zusammensetzung in einer 5 Gew.-%-igen Aufschlämmung in destilliertem Wasser einen pH-Wert der resultierenden Mischung von 4,5 bis 8,0, insbesondere von 5,0 bis 7,5, bewirkt. Diese Ausführungsform eignet sich besonders gut für die Steigerung der Freisetzung von wachsförmigen Estern.

Darüber hinaus zeigte sich, dass die Erfindung besonders gut funktioniert, wenn das partikelförmige, amorphe Metalloxid der pulverförmigen Zusammensetzung eine BET-Oberfläche von 50 bis 1000 m²/g, bevorzugt von 100 bis 750 m²/g, besonders bevorzugt von 150 bis 500 m²/g (jeweils gemessen nach ISO 5794-1), besitzt.

Eine besonders bevorzugte pulverförmige Zusammensetzung umfasst mindestens ein partikelförmiges, amorphes Metalloxid der Ausführungsformen (A) bis (F):
(A):
   Partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1) und
   mit der Maßgabe, dass im besagten Metalloxid mindestens ein kosmetischer Wirkstoff sorbiert vorliegt, der ausgewählt wird unter wachsförmigen Estern.
(B):
   Partikelförmiges, amorphes Metalloxid in Form einer Fällungskieselsäure
      - mit einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid,
   mit der Maßgabe, dass im besagten Metalloxid mindestens ein kosmetischer Wirkstoff sorbiert vorliegt, der ausgewählt wird unter wachsförmigen Estern.
(C):
   Partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1) und
      - einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid,
   mit der Maßgabe, dass im besagten Metalloxid mindestens ein kosmetischer Wirkstoff sorbiert vorliegt, der ausgewählt wird unter wachsförmigen Estern.
(D):
   Partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1) und
      - das in einer 5 Gew.-%-igen Aufschlämmung in destilliertem Wasser einen pH-Wert der resultierenden Mischung von 4,5 bis 8,0, insbesondere von 5,0 bis 7,5, bewirkt,
   mit der Maßgabe, dass im besagten Metalloxid mindestens ein kosmetischer Wirkstoff sorbiert vorliegt, der ausgewählt wird unter wachsförmigen Estern.
(E):
   Partikelförmiges, amorphes Metalloxid in Form einer Fällungskieselsäure
      - mit einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid, und
      - das in einer 5 Gew.-%-igen Aufschlämmung in destilliertem Wasser einen pH-Wert der resultierenden Mischung von 4,5 bis 8,0, insbesondere von 5,0 bis 7,5, bewirkt,
   mit der Maßgabe, dass im besagten Metalloxid mindestens ein kosmetischer Wirkstoff sorbiert vorliegt, der ausgewählt wird unter wachsförmigen Estern.
(F):
   Partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1),
      - einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid, und
      - das in einer 5 Gew.-%-igen Aufschlämmung in destilliertem Wasser einen pH-Wert der resultierenden Mischung von 4,5 bis 8,0, insbesondere von 5,0 bis 7,5, bewirkt,
   mit der Maßgabe, dass im besagten Metalloxid mindestens ein kosmetischer Wirkstoff sorbiert vorliegt, der ausgewählt wird unter wachsförmigen Estern.

Es ist generell bevorzugt und wiederum insbesondere im Rahmen der o.g. bevorzugten Ausführungsformen (insbesondere der Ausführungsformen (A) bis (F)) bevorzugt, wenn der besagte kosmetische Wirkstoff (bzw. Mischungen davon) in einem Gewichtsverhältnisbereich von 1 zu 1 bis 9 zu 1, insbesondere von 3 zu 1 bis 4 zu 1, im partikelförmigen, amorphen Metalloxid sorbiert vorliegt.

Als erfindungsgemäß sorbierter kosmetischer Wirkstoff eignet sich, insbesondere zur Bereitstellung einer pulverförmigen Zusammensetzung zur Verformung keratinhaltiger Fasern, mindestens ein wachsförmiger Ester (im Folgenden auch: Wachsester). Erfindungsgemäße wachsförmige Ester weisen bei 1013 mbar einen Schmelzpunkt in einem Bereich von 40°C bis 95°C auf.

Bevorzugt werden die wachsförmigen Ester aus pflanzlichen, tierischen und mineralischen Wachsen ausgewählt, wobei solche Wachse bevorzugt sind, die einen Schmelzpunkt im Bereich von 40°C bis 90°C, aufweisen.

Erfindungsgemäß besonders bevorzugt, enthält die erfindungsgemäße pulverförmige Zusammensetzung mindestens einen wachsförmigen Ester, ausgewählt aus mindestens einem Wachs der Gruppe Bienenwachs (Cera Alba), Carnaubawachs, Candelillawachs, Montanwachs, Rice Bran Wachs (i.e. Wachs erhältlich aus Reis Kleie), hydrogeniertes Pflanzenöl und Cetylpalmitat.

Die erfindungsgemäße Lehre umfasst auch den kombinierten Einsatz von mehreren Wachsen. So kann ein Zusatz von Carnaubawachs dazu verwendet werden, um Schmelz- und Tropfpunkt eines anderen Wachses zu erhöhen und dessen Klebrigkeit zu vermindern. Diese anderen Wachse können von den wachsförmigen Estern verschieden sein. Bevorzugt sind z.B. mikrokristalline Wachse (insbesondere mikrokristallines Paraffinwachs) und Ozokeritwachs. Entsprechend sind eine Reihe von Wachsmischungen, gegebenenfalls in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für erfindungsgemäß bevorzugt eingesetzte Mischungen.

Es ist im Rahmen aller genannten wachsförmigen Ester (insbesondere der als bevorzugt erwähnten) erfindungsgemäß bevorzugt, für die Sorption des wachsförmigen Esters eines der bevorzugten partikelförmigen, amorphen Metalloxide (insbesondere eines der Ausführungsformen (A) bis (F)) zu verwenden (*vide supra*).

Die erfindungsgemäßen pulverförmigen Zusammensetzungen enthalten die wachsförmigen Ester vorzugsweise in Mengen von 50 Gew.-% bis 90 Gew.-%, bezogen auf das gesamte Mittel. Mengen von 75 Gew.-% bis 80 Gew.-% bezogen auf das gesamte Mittel sind besonders bevorzugt.

Es ist wiederum im Rahmen der wachsesterhaltigen pulverförmigen Zusammensetzungen erfindungsgemäß bevorzugt wenn ein partikelförmiges amorphe Metalloxid vorliegt, auf dessen Partikel in Summe mindestens ein Wachsester und mindestens ein Emulgator sorbiert vorliegt. Im Rahmen dieser Ausführungsform können entweder der Wachsester und der Emulgator gemeinsam auf demselben Partikel des partikelförmigen amorphen Metalloxids sorbiert vorliegen (gemeinsame Sorption) und/oder es handelt sich um eine Mischung von Partikeln eines partikelförmigen amorphen Metalloxids mit sorbiertem Emulgator mit Partikeln eines partikelförmigen amorphen Metalloxids mit sorbiertem wachsförmigen Ester (getrennte Sorption).

Als erfindungsgemäß bevorzugter Emulgator eignet sich mindestens ein nichtionischer Öl-in-Wasser-Emulgator.

Die erfindungsgemäßen pulverförmigen Zusammensetzungen enthalten Emulgatoren - bevorzugt nichtionische ÖI-inWasser-Emulgatoren - vorzugsweise in Mengen von 0,1 Gew.-% bis 20,0 Gew.-%, bezogen auf das gesamte Mittel. Mengen von 1,0 Gew.-% bis 10,0 Gew.-% sind besonders bevorzugt.

Dabei ist es erfindungsgemäß bevorzugt, eine pulverförmige Zusammensetzung der Ausführungsformen (A-I) bis (F-I) einzusetzen:
(A-I):
   Pulverförmige Zusammensetzung, enthaltend mindestens ein partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1) und
   mit der Maßgabe, dass im besagten Metalloxid sorbiert vorliegt:
      - mindestens ein wachsförmiger Ester und
      - mindestens ein nichtionischer Öl-in-Wasser-Emulgator.
(B-I):
   Pulverförmige Zusammensetzung, enthaltend mindestens ein partikelförmiges, amorphes Metalloxid in Form einer Fällungskieselsäure
      - mit einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid,
   mit der Maßgabe, dass im besagten Metalloxid sorbiert vorliegt:
      - mindestens ein wachsförmiger Ester und
      - mindestens ein nichtionischer Öl-in-Wasser-Emulgator.
(C-I):
   Pulverförmige Zusammensetzung, enthaltend mindestens ein partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1) und
      - einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid,
   mit der Maßgabe, dass im besagten Metalloxid sorbiert vorliegt:
      - mindestens ein wachsförmiger Ester und
      - mindestens ein nichtionischer Öl-in-Wasser-Emulgator.
(D-I):
   Pulverförmige Zusammensetzung, enthaltend mindestens ein partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1) und
      - das in einer 5 Gew.-%-igen Aufschlämmung in destilliertem Wasser einen pH-Wert der resultierenden Mischung von 4,5 bis 8,0, insbesondere von 5,0 bis 7,5, bewirkt,
   mit der Maßgabe, dass im besagten Metalloxid sorbiert vorliegt:
      - mindestens ein wachsförmiger Ester und
      - mindestens ein nichtionischer Öl-in-Wasser-Emulgator.
(E-I):
   Pulverförmige Zusammensetzung, enthaltend mindestens ein partikelförmiges, amorphes Metalloxid in Form einer Fällungskieselsäure
      - mit einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid, und
      - das in einer 5 Gew.-%-igen Aufschlämmung in destilliertem Wasser einen pH-Wert der resultierenden Mischung von 4,5 bis 8,0, insbesondere von 5,0 bis 7,5, bewirkt,
   mit der Maßgabe, dass im besagten Metalloxid sorbiert vorliegt:
      - mindestens ein wachsförmiger Ester und
      - mindestens ein nichtionischer Öl-in-Wasser-Emulgator.
(F-I):
   Pulverförmige Zusammensetzung, enthaltend mindestens ein partikelförmiges, amorphes Metalloxid in Form einer Fällungskieselsäure
      - mit einer BET-Oberfläche von 50 bis 1000 m²/g, bevorzugt von 100 bis 750 m²/g, besonders bevorzugt von 150 bis 500 m²/g (jeweils gemessen nach ISO 5794-1),
      - einer DBP-Zahl von 50 bis 800 g, bevorzugt von 100 bis 500 g, besonders bevorzugt von 200 bis 400 g, ganz besonders bevorzugt von 250 bis 350 g, jeweils pro 100 g Metalloxid, und
      - das in einer 5 Gew.-%-igen Aufschlämmung in destilliertem Wasser einen pH-Wert der resultierenden Mischung von 4,5 bis 8,0, insbesondere von 5,0 bis 7,5, bewirkt,
   mit der Maßgabe, dass im besagten Metalloxid sorbiert vorliegt:
      - mindestens ein wachsförmiger Ester und
      - mindestens ein nichtionischer Öl-in-Wasser-Emulgator.

Im Rahmen der bevorzugten Ausführungsformen (A-I) bis (F-I) sind wiederum die obigen als bevorzugt gekennzeichneten wachsförmigen Ester und/oder die bevorzugten Einsatzmengen bevorzugt geeignet. Gleiches gilt für die nachfolgend als bevorzugt geeigneten nichtionischen Emulgatoren und/oder deren bevorzugte Einsatzmengen.

Bevorzugte erfindungsgemäße kosmetische Zusammensetzungen sind dadurch gekennzeichnet, dass die Emulgatoren aus mindestens einer Verbindung der Gruppe ausgewählt werden, die gebildet wird aus ethoxylierten C₈-C₂₄-Alkanolen mit durchschnittlich 8 - 100 Mol Ethylenoxid pro Mol, ethoxylierten C₈-C₂₄-Carbonsäuren mit durchschnittlich 8 - 100 Mol Ethylenoxid pro Mol, mit durchschnittlich 20 - 100 Mol Ethylenoxid pro Mol ethoxylierten Glycerinmono- und diestern von linearen gesättigten und ungesättigten C₁₂ - C₃₀-Carbonsäuren, die hydroxyliert sein können, insbesondere diejenigen von Myristinsäure, Palmitinsäure, Stearinsäure, 12-Hydroxystearinsäure oder von Mischungen dieser Fettsäuren, mit durchschnittlich 20 - 100 Mol Ethylenoxid pro Mol, ethoxylierten Sorbitanmonoestern von linearen gesättigten und ungesättigten C₁₂ - C₃₀-Carbonsäuren, die hydroxyliert sein können, insbesondere diejenigen von Myristinsäure, Palmitinsäure, Stearinsäure, 12-Hydroxystearinsäure oder von Mischungen dieser Fettsäuren, Silicon-Copolyolen mit Ethylenoxid-Einheiten oder mit Ethylenoxid- und Propylenoxid-Einheiten, Alkylmono- und - oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, sofern letztere einen HLB-Wert von mehr als 7 aufweisen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 8 - 100, vorzugsweise 8 - 30 Mol Ethylenoxid an 1 Mol Caprylalkohol, 2-Ethylhexylalkohol, Caprinalkohol, Laurylalkohol, Isotridecylalkohol, Tridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 8 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.

Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 8 - 100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 8 - 30 Einheiten Ethylenoxid pro Molekül sowie Mischungen dieser Substanzen, insbesondere Laureth-8, Laureth-10, Laureth-12, Laureth-20, Trideceth-8, Trideceth-9, Trideceth-10, Trideceth-12, Trideceth-20, Ceteth-10, Ceteth-12, Ceteth-20, Ceteth-30, Steareth-10, Steareth-12, Steareth-20, Steareth-30, Ceteareth-10, Ceteareth-12, Ceteareth-20, Ceteareth-30, Laureth-12 und Beheneth-20.

Bevorzugte mit durchschnittlich 20 - 100 Mol Ethylenoxid pro Mol ethoxylierte Glycerinmono- und/oder diester von linearen gesättigten und ungesättigten C₁₂ - C₃₀-Carbonsäuren, die hydroxyliert sein können, sind ausgewählt aus PEG-20 Hydrogenated Castor Oil, PEG-40 Hydrogenated Castor Oil und PEG-60 Hydrogenated Castor Oil.

Bevorzugte mit durchschnittlich 20 - 100 Mol Ethylenoxid pro Mol ethoxylierte Sorbitanmonoester von linearen gesättigten und ungesättigten C₁₂ - C₃₀-Carbonsäuren, die hydroxyliert sein können, sind ausgewählt aus Polysorbate-20, Polysorbate-40, Polysorbate-60 und Polysorbate-80.

Weiterhin werden vorzugsweise C₈ - C₂₂-Alkylmono- und -oligoglycoside eingesetzt. C₈ -C₂₂-Alkyl- mono- und -oligoglycoside stellen bekannte, handelsübliche Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 - 1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Im Rahmen aller vorgenannten Ausführungsformen der Erfindung erwies es sich als besonders vorteilhaft, insbesondere für die Anwendung der erfindungsgemäßen pulverförmigen Zusammensetzungen auf trockenem Substrat, der erfindungsgemäßen pulverförmigen Zusammensetzung zusätzlich Kern-Hülle-Teilchen zuzusetzen, deren Hülle Partikel mindestens eines hydrophobierten Metalloxidpulvers enthält und deren Kern eine flüssige, wässrige Phase umfasst.

Die Kern-Hülle-Teilchen der pulverförmigen Zusammensetzung gemäß obiger Ausführungsform der vorliegenden Erfindung umfassen einen Kern, der eine wässrige, flüssige Phase umfasst. Der Kern liegt somit in flüssiger Form vor. Diesen Kern umgibt eine Hülle, die auf separierbaren einzelnen Partikeln mindestens eines hydrophobierten Metalloxidpulvers basiert.

Die Art des hydrophobierten Metalloxids ist nicht prinzipiell beschränkt, solange sichergestellt ist, dass beim intensiven Vermischen mit der flüssigen, wässrigen Phase ein entsprechendes Kern-Hülle-Teilchen entsteht. Unter hydrophobiert sind im Sinne der Erfindung solche Metalloxide zu verstehen, die zumindest an der Oberfläche der Partikel derart modifiziert wurden, dass das modifizierte Teilchen weniger von Wasser benetzt wird als das nicht modifizierte Teilchen. Insbesondere sind silanisierte, hydrophobierte Metalloxide bevorzugt. Als Reagenz zur Silanisierung des Matalloxids eignet sich erfindungsgemäß bevorzugt mindestens ein Vertreter der Gruppe, die gebildet wird, aus Silanen, Halogensilanen, Alkoxysilanen und Silazanen.

Bevorzugt geeignete hydrophobierte Metalloxide des hydrophobierten Metalloxidpulvers werden erfindungsgemäß ausgewählt aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid.

Besonders bevorzugte Aluminiumsilikate (auch Alumosilikate genannt) werden ausgewählt unter Phyllosilikaten, Tectosilikaten oder Gemischen daraus.

Bevorzugt geeignete Phyllosilikate werden ausgewählt unter Kaolinen (hier insbesondere unter Kaolinit, Dickit, Hallosit sowie Nakrit), Serpentin, Talk, Pyrophyllit, Montmorillonit, Quarz, Bentonit, Glimmer (hier insbesondere unter Illit, Muscovit, Paragonit, Phlogopit, Biotit, Lepidolith, Margarit, Smektit (hier insbesondere unter Montmorrilionit, Saponit, Nontronit, Hectorit)).

Bevorzugt geeignete Tectosilikate werden ausgewählt unter Feldspatmineralien (insbesondere Albit, Orthoklas, Anorthit, Leucit, Sodalith, Hauyn, Labradorit, Lasurit, Nosean, Nephelin), Zeolithen.

Die Kern-Hülle-Teilchen der erfindungsgemäß bevorzugten pulverförmigen Zusammensetzungen enthalten das hydrophobierte Metalloxidpulver vorzugsweise in einer Menge von 0,5 bis 30 Gew.-%, bezogen auf das Gewicht der in der erfindungsgemäßen pulverförmigen Zusammensetzung enthaltenen Kern-Hülle-Teilchen.

Ferner hat es sich als bevorzugt erwiesen, wenn die hydrophobierten Metalloxide einen Partikeldurchmesser von weniger als 5 µm, bevorzugt weniger als 1 µm, besonders bevorzugt zwischen 20 und 100 nm, aufweisen.

Besonders bevorzugt enthält die erfindungsgemäße pulverförmige Zusammensetzung als hydrophobiertes Metalloxidpulver der Kern-Hülle-Teilchen mindestens ein hydrophobiertes Siliziumdioxid, insbesondere mindestens ein silanisiertes, hydrophobiertes Siliziumdioxid.

Als Reagenz zur Silanisierung des Siliziumdioxids eignen sich erfindungsgemäß bevorzugt mindestens ein Vertreter der Gruppe, die gebildet wird, aus Silanen, Halogensilanen, Alkoxysilanen und Silazanen.

Bevorzugte Vertreter der Gruppe der Silane sind Hexa(C₁-C₂₀)alkyldisilane, insbesondere Hexamethyldisilan.

Wenn ein Halogensilan als Silylierungsmittel Anwendung findet, wird als bevorzugtes Halogensilan mindestens eine Verbindung aus der Gruppe ausgewählt, die gebildet wird, aus den Verbindungen

[(C₁-C₂₀)Alkyl]_{z'}SiX_{(4-z')}

X₃Si[(CH₂)ₙ-R]

X₂[(C₁-C₂₀)Alkyl]Si(CH₂)ₙ-R

[(C₁-C₂₀)Alkyl]_{(y'+1)}[R-(CH₂)ₙ]_{(2-y')}SiX

worin
X ein Chlor-, Brom- oder lodatom bedeutet,
z' eine Zahl 1, 2 oder 3 ist,
y' eine Zahl 0, 1 oder 2 ist
n eine ganze Zahl von 1 bis 20 ist und
R steht für einen Rest aus
(C₁-C₁₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 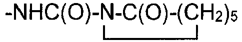 -NH-C(O)O-Me, -NH-C(O)O-Et, -NH-(CH₂)₃-Si(O(C₁-C₆)alkyl)₃.

Wenn ein Alkoxysilan als Silylierungsmittel verwendet wird, wird als bevorzugtes Alkoxysilan mindestens eine Verbindung aus der Gruppe ausgewählt, die gebildet wird, aus den Verbindungen

[(C₁-C₂₀)AlkylO]_{z}Si(C₁-C₂₀)Alkyl_{(4-z)}

[(C₁-C₂₀)AlkylO]_{z}Si[(CH₂)ₙ-R]_{(4-z)}

[(C₁-C₂₀)AlkylO]₂[(C₁-C₂₀)Alkyl]Si(CH₂)ₙ-R

[(C₁-C₂₀)AlkylO][(C₁-C₂₀)Alkyl]₂Si(CH₂)ₙ-R

[(C₁-C₂₀)AlkylO][(C₁-C₂₀)Alkyl]Si[(CH₂)ₙ-R]₂

(C₁-C₂₀Alkyl)₃SiO-C(CH₃)=N-Si(C₁-C₂₀)Alkyl₃,

worin
n eine ganze Zahl von 1 bis 20 ist und
z eine Zahl 1, 2, oder 3 bedeutet
R steht für einen Rest aus
(C₁-C₂₀)Alkyl-, Aryl-, (C₁-C₆)Perfluoroalkyl-, -NH₂, -N₃, -SCN, -CH=CH₂, -O(O)C-C(CH₃)=CH₂, -OCH₂-CH=CH₂, 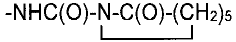 -NH-C(O)O-Me, -NH-C(O)O-Et, -NH-(CH₂)₃-Si(O(C₁-C₆)alkyl)₃.

Als bevorzugtes Silazan wird mindestens eine Verbindung aus der Klasse der Disilazane, insbesondere mindestens eine Verbindung aus Disilazanen der Formel

R'₂R"Si-NH-SiR'₂R"

ausgewählt, worin
R' eine (C₁-C₂₀)Alkylgruppe bedeutet und
R" eine (C₁-C₂₀)Alkylgruppe oder eine Vinylgruppe bedeutet. Ein besonders bevorzugtes Silazan ist Hexamethyldisilazan.

Alle oben genannten Alkylgruppen, ob (C₁-C₆)Alkyl, (C₁-C₁₀)Alkyl oder (C₁-C₂₀)-Alkyl, können sowohl zyklisch, als auch linear bzw. verzweigt sein. Beispiele für erfindungsgemäß verwendbare Alkylgruppen sind Methyl, Ethyl, n-Propyl, Isopropyl, n-Butyl, Cyclopentyl, Cyclohexyl, n-Decyl, Lauryl, Myristyl, Cetyl, Stearyl, Isostearyl und Behenyl.

Ein Beispiel für eine erfindungsgemäße Arylgruppe ist die Phenylgruppe.

Beispiele für eine erfindungsgemäße (C₁-C₆)Perfluoroalkylgruppe sind Trifluormethyl, Perfluoroethyl, Perfluoropropyl und Perfluorohexyl.

Vorzugsweise werden hydrophobierte Siliziumdioxide eingesetzt, die durch Silanisierung von pyrogenem Siliziumdioxid erhalten werden.

Silanisierte, hydrophobierte Siliziumdioxide werden insbesondere bevorzugt ausgewählt aus mindestens einer Verbindung der Gruppe, die gebildet wird aus Trimethylsilylat-beschichtetem Siliziumdioxid, Dimethylsilylat-beschichtetem Siliziumdioxid, Octylsilylat-beschichtetem Siliziumdioxid.

Dabei ist es wiederum bevorzugt das hydrophobierte Metalloxidpulver der Kern-Hülle-Teilchen aus Silica Silylate auszuwählen. Dies sind hydrophobierte Siliziumdioxide, die der INCl-Bezeichnung Silica Silylate gehorchen.

Bevorzugt sind solche hydrophobierten Siliziumdioxide, die eine spezifische Oberfläche nach BET zwischen 10 und 400 m²/g, vorzugsweise zwischen 80 bis 300 m²/g aufweisen. Dabei sind wiederum solche hydrophobierte Siliziumdioide bevorzugt geeignet, die silanisiert sind, insbesondere Silica Silylat.

Eine Vielzahl geeigneter hydrophobierter Siliziumdioxide ist kommerziell erhältlich. Beispielhaft seien Aerosil^{®} R104 V, Aerosil^{®} R106, Aerosil^{®} R202, Aerosil^{®} R805, Aerosil^{®} R812, Aerosil^{®} R812S, Aerosil^{®} R972 und Aerosil^{®} R8200, alle Degussa, sowie HDK^{®} H2000, HDK^{®} H2050 und HDK^{®} H3004, alle Wacker, genannt.

Besonders bevorzugt werden die hydrophobierten Siliziumdioxide eingesetzt, die unter den Bezeichnungen Aerosil^{®} R202, Aerosil^{®} R812S oder Aerosil^{®} R972 erhältlich sind. Ganz besonders bevorzugt kommt das Siliziumdioxid mit der INCI-Bezeichnung Silica Silylate zum Einsatz, das von der Firma Degussa unter der Bezeichnung Aerosil^{®} R812S vertrieben wird.

Die Kern-Hülle-Teilchen der erfindungsgemäß bevorzugten pulverförmigen Zusammensetzungen enthalten das hydrophobierte Siliziumoxidpulver vorzugsweise in einer Menge von 0,5 bis 30 Gew.-%, bezogen auf das Gewicht der in der erfindungsgemäßen pulverförmigen Zusammensetzung enthaltenen Kern-Hülle-Teilchen. Dabei ist es wiederum besonders bevorzugt, das hydrophobierte Siliziumdioxidpulver aus Silica Silylate auszuwählen und in einer Menge von 0,5 bis 30 Gew.-%, bezogen auf das Gewicht der in der erfindungsgemäßen pulverförmigen Zusammensetzung enthaltenen Kern-Hülle-Teilchen einzusetzen. Die optimale Menge hängt dabei vor allem von der Hydrophobizität des eingesetzten Siliziumdioxidpulvers ab. Je hydrophober das Siliziumdioxidpulver ist, desto weniger davon wird benötigt, um ein stabiles, pulverförmiges Produkt zu erhalten.

Bevorzugt enthält die erfindungsgemäße pulverförmige Zusammensetzung als hydrophobiertes Metalloxid mindestens ein hydrophobiertes Phyllosilikat, insbesondere mindestens einen hydrophobierten Glimmer und/oder mindestens einen hydrophobierten Talk. Dabei eignet sich wiederum mindestens ein silanisiertes, hydrophobiertes Phyllosilikat bzw. mindestens ein silanisierter hydrophobierter Glimmer und/oder mindestens ein hydrophobierter Talk bevorzugt.

Als Reagenz zur Silanisierung des Phyllosilikats bzw. des Glimmers eignen sich erfindungsgemäß bevorzugt mindestens ein Vertreter der Gruppe, die gebildet wird, aus Silanen, Halogensilanen, Alkoxysilanen und Silazanen. Für die besonders bevorzugten Reagenzien wird *mutatis mutandis* auf die Ausführungen zur Silanisierung des Siliziumdioxids verwiesen (vide *supra*).

Silanisierte, hydrophobierte Glimmer werden insbesondere bevorzugt ausgewählt aus mindestens einer Verbindung der Gruppe, die gebildet wird aus Trimethylsilylat-beschichtetem Glimmer, Dimethylsilylat-beschichtetem Glimmer, Octylsilylat-beschichtetem Glimmer.

Silanisierter, hydrophobierter Talk wird insbesondere bevorzugt ausgewählt aus mindestens einer Verbindung der Gruppe, die gebildet wird aus Trimethylsilylat-beschichtetem Talk, Dimethylsilylat-beschichtetem Talk, Octylsilylat-beschichtetem Talk.

Als erfindungsgemäß einsetzbares hydrophob modifiziertes Phyllosilikat sind beispielsweise mit Triethoxycaprylsilan silanisierter, hydrophobierter Glimmer mit Triethoxycaprylsilan silanisierter, hydrophobierter Talk beispielsweise von der Firma LCW zu nennen.

Ferner enthalten die Kern-Hülle-Teilchen der erfindungsgemäß bevorzugten pulverförmigen Zusammensetzungen an der Oberfläche mit fluorhaltigen, organischen Gruppen beschichtete Metalloxidpartikel als hydrophobiertes Metalloxidpulver (insbesondere an der Oberfläche mit fluorhaltigen, organischen Gruppen beschichtetem Glimmer, an der Oberfläche mit fluorhaltigen, organischen Gruppen beschichtetem Titandioxid, an der Oberfläche mit fluorhaltigen, organischen Gruppen beschichtetem Siliziumdioxid) enthalten. Besonders zur Ausführung dieser Ausführungsform geeignete an der Oberfläche mit fluorhaltigen, organischen Gruppen beschichtete Metalloxidpartikel sind solche, die an der Oberfläche mit perfluorierten organischen Verbindungen, insbesondere mit Perfluoralkylresten, beschichtet wurden. Dabei sind wiederum solche besagten Metalloxide bevorzugt, die mit mindestens einem Rest hydrophobiert wurden, ausgewählt aus Perfluoralkylsilyl, Polyperfluor-(C₂ bis C₆)-alkylenoxid, Polysiloxan mit Perfluoralkylgruppen, Perfluoralkylphosphat, Polyfluoralkylphosphatether. Darunter werden solche an der Oberfläche mit fluorhaltigen, organischen Gruppen beschichteten Metalloxidpartikel bevorzugt verwendet, die an der Oberfläche Polyperfluor-(C₂ bis C₆)-alkylenoxidreste tragen. Diese werden bevorzugt ausgewählt aus mit Polyperfluor-(C₂ bis C₆)-alkylenoxid beschichtetem Glimmer und/oder mit Polyperfluor-(C₂ bis C₆)-alkylenoxid beschichtetem Titandioxid und/oder mit Polyperfluor-(C₂ bis C₆)-alkylenoxid beschichtetem Siliziumdioxid und/oder mit Polyperfluor-(C₂ bis C₆)-alkylenoxid beschichtetem Talk.

Besonders bevorzugt einsetzbar sind im Rahmen dieser Ausführungsform dabei die Handelsprodukte PW Covafluor^{®} (Firma LCW), PFS Talc JA 46-R^{®} (Firma Daito), Talc JA R46PF^{®} (Firma LCW), Submica M^{®} (Firma LCW).

Ganz besonders bevorzugt eignen sich im Rahmen dieser Ausführungsform solche an der Oberfläche mit fluorhaltigen, organischen Gruppen beschichtete Metalloxidpartikel, die an der Oberfläche durch Umsetzung mit mindestens einem Reagenz der Formel (I) beschichtet wurden 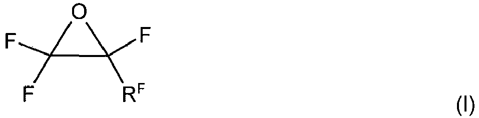 worin R^{F} für eine Perfluor-(C₁ bis C₄)-alkylgruppe, insbesondere Trifluormethyl, steht.

Metalloxidpartikel, die mit Reagenzien der Formel (I) beschichtet wurden erhalten kovalent an der Oberfläche gebundene Polyperfluor-(C₂ bis C₆)-alkylenoxidreste.

Darüber hinaus ist es im Rahmen der besagten Ausführungsform erfindungsgemäß bevorzugt, wenn die an der Oberfläche mit fluorhaltigen, organischen Gruppen beschichteten Metalloxidpartikel durch mindestens eine Verbindung der Formel (la) beschichtet sind 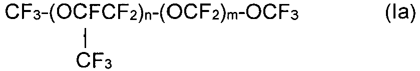 worin die Summe n + m für eine ganze Zahl von 20 bis 80 steht. Diese Verbindungen besitzen die INCl-Bezeichnung Polyperfluoromethylisopropyl Ether. Besonders bevorzugt eignen sich Verbindungen der Formel (Ia), die ein Molekulargewicht von 1500 bis 4000 g/mol aufweisen.

Ganz besonders bevorzugt einsetzbar ist das Handelsprodukt PW F-MS (mit Polyperfluoromethylisopropylether (CAS-Nr.: 69991-67-9, EINECS 274-225-4) und Triethoxycaprylylsilan beschichtetes, nanofeines Titandioxid (INCI-Bezeichnung: Cl 77891, Polyperfluoromethylisopropyl Ether, Triethoxycaprylylsilane) der Firma Sensient / LCW.

Unter einer flüssigen, wässrigen Phase wird eine Flüssigkeit verstanden, die mindestens 40 Gew.-%, insbesondere mindestens 65 Gew.-%, Wasser bezogen auf das Gewicht der flüssigen, wässrigen Phase im Kern, enthält.

Die flüssige, wässrige Phase der Kern-Hülle-Teilchen der bevorzugten Ausführungsform der pulverförmigen Zusammensetzung enthält dabei bevorzugt ein wässriges Lösungsmittel ausgewählt aus Wasser oder einem Gemisch aus Wasser und einem C₁-C₄-Alkohol, insbesondere Ethanol. Da oberflächenaktive Substanzen und Alkohole unter Umständen jedoch hydrophobiertes Siliziumdioxid benetzen und damit die hydrophoben Eigenschaften negativ beeinflussen können, kann es je nach Art des eingesetzten hydrophobierten Siliziumdioxids notwendig sein, den Gehalt an C₁-C₄-Alkohol im wässrigen Lösungsmittel unter einer kritischen Maximalmenge zu halten.

Vorzugsweise enthalten die Kern-Hülle-Teilchen in der flüssigen, wässrigen Phase bezogen auf das Gewicht der Kern-Hülle-Teilchen der pulverförmigen Zusammensetzung 70 Gew.-% bis 90 Gew.-%, besonders bevorzugt 80 bis 90 Gew.-%, eines wässrigen Lösungsmittels.

Vorzugsweise wird daher als wässriges Lösungsmittel Wasser oder ein Gemisch aus Wasser und maximal 60 Gew.-% C₁-C₄-Alkohol, bezogen auf das Lösungsmittelgemisch, eingesetzt. Besonders bevorzugte wässrige Lösungsmittel sind Wasser oder ein Gemisch aus Wasser und maximal 30 Gew.-% C₁-C₄-Alkohol, bezogen auf das Lösungsmittelgemisch. Ganz besonders bevorzugt wird als wässriges Lösungsmittel Wasser eingesetzt.

Die Kern-Hülle-Teilchen der bevorzugten Ausführungsform der pulverförmigen Zusammensetzung sind dazu geeignet, die flüssige, wässrige Phase des Kerns durch mechanische Belastung der Kern-Hülle-Teilchen, insbesondere durch Reibung und/oder Druck, aus dem Kern-Hülle-Teilchen freizusetzen und dabei aus der pulverförmigen Zusammensetzung eine Flüssigkeit zu bilden.

Die pulverförmigen Zusammensetzungen können in nahezu beliebigen Behältnissen konfektioniert werden. Falls die erfindungsgemäße Zusammensetzung Kern-Hülle-Teilchen enthält, muss lediglich sichergestellt werden, dass die mechanische Belastung des Pulvers bei der Entnahme der Zusammensetzung nicht so hoch ist, dass bereits bei der Entnahme das Pulver in flüssige Form überführt wird. Geeignet sind beispielsweise Tiegel, Flaschen oder auch Tetrapacks, wobei das Behältnis beispielsweise mit einer Schütt- und Dosiervorrichtung ausgestaltet werden kann.

Alle erfindungsgemäßen pulverförmigen Zusammensetzungen können zumindest durch folgendes bevorzugtes Herstellungsverfahren bereitgestellt werden, welches der zweite Gegenstand der Erfindung ist:
Die partikelförmigen, amorphen Metalloxide, enthaltend mindestens einen erfindungsgemäßen, sorbierten Wirkstoff, werden beispielsweise wie folgt hergestellt: Der besagte kosmetische Wirkstoff wird in flüssiger Form (erzielt entweder durch auflösen in einem Lösemittel oder durch schmelzen) mit dem partikelförmigen, amorphen Metalloxid in einem Mischer (z.B. Propellermischer) zunächst aufgeschlämmt und dann solange verrührt, bis das partikelförmige, amorphe Metalloxid den besagten kosmetischen Wirkstoff aufgenommen hat und eine pulverförmige Zusammensetzung resultiert. Eine andere Herstellungsmethode ist die innige Vermengung eines festförmigen Sorbats mit dem besagten amorphen Metalloxid als Sorbens z.B. in einem Mörser oder Mahlwerk.

Die Kern-Hülle-Teilchen als Zusatz im Rahmen der bevorzugten Ausführungsform der erfindungsgemäßen pulverförmigen Zusammensetzung lassen sich einfach herstellen. Es hat sich bei Einsatz unterschiedlicher erfindungsgemäß beschichteter Metalloxidpartikel bewährt, zunächst alle erfindungsgemäß beschichteten Metalloxidpartikel zu vermischen. Dann werden in einem separaten Mischer alle Inhaltsstoffe bis auf besagte erfindungsgemäß beschichtete Metalloxidpartikel in die wässrige Phase unter Rühren eingearbeitet. Schließlich werden die beschichteten Metalloxidpartikel unter intensivem Rühren zu der wässrigen Phase gegeben. Die benötigte Mischzeit ist abhängig von der eingebrachten Mischenergie und der jeweiligen Zusammensetzung der Mischung, beträgt aber in der Regel zwischen 15 Sekunden und 5 Minuten. Wird zu kurz gemischt, bildet sich kein stabiles Pulver aus und es kommt zur Ausbildung einer öligen Phase. Bei zu langer Mischzeit wird das zunächst entstehende Pulver in eine brei- oder cremeartige Konsistenz überführt, wobei dieser Vorgang nicht reversibel verläuft. Es empfiehlt sich daher, durch einige Vorversuche die optimale Mischzeit für das jeweilige System zu ermitteln.

Eine erfindungsgemäß bevorzugte pulverförmige Zusammensetzung umfassend Kern-Hülle-Teilchen wird beispielsweise durch vorsichtiges vermengen der Kern-Hülle-Teilchen mit dem besagten sorbathaltigen, pulverförmigen, amorphen Metalloxid bereitgestellt.

Die erfindungsgemäßen pulverförmigen Zusammensetzungen werden zur temporären Verformung keratinhaltiger Fasern, insbesondere menschlicher Haare verwendet.

Unter keratinhaltigen Fasern sind dabei erfindungsgemäß Pelze, Wolle, Federn und insbesondere menschliche Haare zu verstehen.

Ein vierter Erfindungsgegenstand ist ein Verfahren zur kosmetischen Behandlung keratinhaltiger Fasern, insbesondere menschlicher Haare, in dem
a) die keratinhaltigen Fasern gegebenenfalls angefeuchtet werden,
b) eine pulverförmige Zusammensetzung enthaltend
   - mindestens eine partikelförmige, amorphe Fällungskieselsäure, mit der Maßgabe, dass in besagter Fällungskieselsäure mindestens ein kosmetischer Wirkstoff sorbiert vorliegt, der ausgewählt wird unter wachsförmigen Estern;
   - Kern-Hülle-Teilchen, deren Hülle Partikel mindestens eines hydrophobierten Metalloxidpulvers enthält und deren Kern eine flüssige, wässrige Phase umfasst auf die keratinhaltigen Fasern aufgetragen wird
c) diese pulverförmige Zusammensetzung während oder nach dem Auftragen auf die keratinhaltigen Fasern einer mechanischen Belastung ausgesetzt wird, wobei sich die pulverförmige Zusammensetzung umfassend besagte Kern-Hülle-Teilchen in eine Flüssigkeit umwandelt,
d) die aufgetragene Zusammensetzung auf die keratinhaltigen Fasern einwirkt und gegebenenfalls ausgespült wird.

Bevorzugt ist ein Verfahren zur Verformung keratinhaltiger Fasern, insbesondere menschlicher Haare, in dem
a) die keratinhaltigen Fasern gegebenenfalls angefeuchtet werden,
b) eine pulverförmige Zusammensetzung enthaltend
   - mindestens eine partikelförmige, amorphe Fällungskieselsäure, mit der Maßgabe, dass in besagter Fällungskieselsäure mindestens ein kosmetischer Wirkstoff sorbiert vorliegt, der ausgewählt wird unter wachsförmigen Estern;
   - Kern-Hülle-Teilchen, deren Hülle Partikel mindestens eines hydrophobierten Metalloxidpulvers enthält und deren Kern eine flüssige, wässrige Phase umfasst auf die keratinhaltigen Fasern aufgetragen wird
c) diese pulverförmige Zusammensetzung während oder nach dem Auftragen auf die keratinhaltigen Fasern einer mechanischen Belastung ausgesetzt wird, wobei sich die pulverförmige Zusammensetzung umfassend besagte Kern-Hülle-Teilchen in eine Flüssigkeit umwandelt,
d) die keratinhaltigen Fasern
   - vor, nach oder während des Auftragens der pulverförmigen Zusammensetzung und/oder
   - vor, nach oder während der mechanischen Belastung der pulverförmigen Zusammensetzung
   in Form gebracht werden,
e) die Form der keratinhaltigen Fasern durch die besagte pulverförmige Zusammensetzung temporär fixiert wird.

Im Rahmen dieser Ausführungsform des Verfahrens ist es bevorzugt, die Zusammensetzung nicht von der Faser zu spülen (Einsatz als leave-on Kosmetikum).

Im Sinne des o.g. Verfahrens wird zunächst bevorzugt die gewünschte Menge der pulverförmigen Zusammensetzung dem Behältnis entnommen. Die Zusammensetzung kann direkt auf die zu behandelnde keratinische Faser oder aber beispielsweise auf die Hand gegeben werden.

Die pulverförmige Zusammensetzung lässt sich direkt aus dem Behältnis auf die Fasern applizieren, aber natürlich auch mit einem Hilfsmittel, etwa der Hand, einem Pinsel, einem Schwamm, einem Tuch, einer Bürste oder einem Kamm. Die Hilfsmittel können auch dazu verwendet werden, die aufgetragene Zusammensetzung auf der Faser zu verteilen.

Umfasst die pulverförmige Zusammensetzung besagte Kern-Hülle-Teilchen, kann das aufgebrachte Pulver direkt auf der keratinischen Faser einer mechanischen Belastung, beispielsweise mittels der Hände oder Hilfsmitteln (*vide supra*), ausgesetzt werden, wodurch die flüssige, wässrige Phase in der pulverförmigen Zusammensetzung enthaltener Kern-Hülle-Teilchen direkt auf der Faser freigesetzt und die Wirkung des sorbierten Wirkstoffs entfaltet werden. Wird die pulverförmige Zusammensetzung umfassend Kern-Hülle-Teilchen zunächst auf die Hand gegeben, so kann sie zunächst vorsichtig im Haar verteilt und wiederum erst anschließend stärker mechanisch belastet werden, beispielsweise durch gezieltes Einmassieren des Pulvers in das Haar. Dadurch wird die flüssige, wässrige Phase der Kern Hülle Teilchen direkt auf der Faser freigesetzt und die Wirkung des sorbierten Wirkstoffs entfaltet. So lässt sich sehr gezielt eine hervorragende kosmetische Wirkung erreichen. Es ist natürlich auch möglich, die pulverförmige Zusammensetzung bereits auf der Hand zu verreiben und erst das entstehende flüssige oder pastenartige Mittel auf die keratinische Faser aufzubringen. Diese Vorgehensweise ist allerdings nicht bevorzugt, da dabei auf einen wesentlichen Vorteil der pulverförmigen Konsistenz der Zusammensetzung, nämlich die gute Verteilbarkeit, verzichtet wird.

Die nachfolgenden Beispiele sollen den Gegenstand der vorliegenden Erfindung erläutern ohne ihn in irgendeiner Weise zu beschränken.

### Beispiele

Die im Folgenden angegebenen Mengenangaben verstehen sich - soweit nichts anderes vermerkt ist - in Gewichtsprozent.

| **Rohstoff** | **E1** | **E2** | **E3** | **E4** | **E5** | **E6** | **E7** | **E8** | **E9** | **E10** |
|---|---|---|---|---|---|---|---|---|---|---|
| Phenoxyethanol, rein | 0,6 | 0,6 | 0,6 | 1,0 | 1,0 | 0,6 | 0,6 | 0,6 | 0,6 | 0,6 |
| Wasser, vollentsalzt | 6,4 | 6,4 | 6,4 | - | - | 6,4 | - | - | - | - |
| Isopropylmyristat | 5,0 | - | - | - | - | - | - | - | - | - |
| Wachsemulsion 2168/5BWCW | 7,0 | 7,0 | - | - | - | - | - | - | - | 7,0 |
| Sipernat^{®} 500LS | 10,0 | 25,0 | 25,0 | 24,0 | 20,0 | 22,5 | 22,0 | 21,0 | 18,5 | 16,4 |
| Aerosil^{®} R 812 S | 1,0 | - | - | - | - | - | - | - | - | - |
| Dow Corning^{®} 193 C Fluid | - | 5,0 | 5,0 | 5,0 | 5,0 | 5,0 | 5,0 | 5,0 | 5,0 | 5,0 |
| Luviskol^{®} VA 64 W | - | - | - | - | 5,0 | - | - | - | - | - |
| Kern-Hülle-Teilchen | - | - | - | - | - | 2,5 | 2,0 | 1,5 | 1,5 | 1,0 |
| PEG-45M | - | - | - | - | - | - | 0,4 | 2,0 | 2,0 | - |
| Sorbitol | - | - | - | - | - | - | 3,5 | 3,5 | 3,5 | - |
| Verityl AB-1000 Liquid | - | - | - | - | - | - | 2,0 | - | - | - |
| Glycerin | <------------------------------------ ad 100------------------------------------ > | | | | | | | | | |

| **Rohstoff** | **E11** | **E12** | **E13** | **E14** | **E15** | **E16** | **E17** | **E18** | **E19** | **E20** |
|---|---|---|---|---|---|---|---|---|---|---|
| Phenoxyethanol, rein | 0,6 | 0,6 | 0,6 | 0,6 | 0,6 | 0,6 | 0,6 | 0,6 | 0,6 | 0,6 |
| Kahlwax 6240 Vegetable Wax | 10,0 | 10,0 | 5,0 | 5,0 | 5,0 | 5,0 | 10,0 | 10,0 | 10,0 | 5,0 |
| Bienenwachs | - | 10,0 | - | - | - | - | - | - | - | - |
| Dow Corning 9701 Cosmetic Powder | - | - | - | - | - | - | - | - | - | 2,0 |
| PEG-32 | 10,0 | 10,0 | 5,0 | 5,0 | 5,0 | 5,0 | 5,0 | 10,0 | 5,0 | - |
| PEG-8 | 10,0 | 15,0 | - | - | - | - | - | - | - | - |
| PEG-40 Hydrogenated Castor Oil | - | - | 5,0 | 5,0 | 5,0 | 5,0 | 5,0 | - | 5,0 | - |
| Cocoglycerides + 7.3 EO | - | 15,0 | - | - | - | - | - | - | - | - |
| Sipernat^{®} 2200 | - | - | 14,4 | - | - | - | - | - | - | - |
| Sipernat^{®} 22 LS | - | - | - | 14,4 | - | - | - | - | - | - |
| Sipernat^{®} 50 | - | - | - | - | 14,4 | - | - | - | - | - |
| Sipernat^{®} 500LS | 23,0 | 23,0 | - | - | - | 25,0 | 23,0 | 23,0 | 23,0 | 22,4 |
| Wachsemulsion 2168/5BWCW | - | - | - | - | - | - | - | 7,0 | - | - |
| Polyvinylpyrrolidon | - | - | - | - | - | - | - | - | 1,4 | - |
| Glycerin | < ------------------------------------ad 100------------------------------------ > | | | | | | | | | |

### 1. Herstellung der pulverförmigen Stylingmittel

Es wurden wie nachfolgend beschrieben die erfindungsgemäßen pulverförmigen Stylingmittel E1 bis E19 hergestellt.

Alle Rohstoffe bis auf Sipernat, Aerosil bzw. die Aerosil-haltige Mischung 1 wurden miteinander gemischt, wobei nötigenfalls feste Rohstoffe aufgeschmolzen wurden. Die resultierende Mischung wurde mit Sipernat und ggf. Aerosil vermengt, bis die Mischung durch das Sipernat und ggf. Aerosil sorbiert wurde und eine pulverförmige Zusammensetzung entstand. Die pulverförmige Mischung 1 wurde sodann - falls in Rezeptur vorhanden - vorsichtig unter das zuvor hergestellte Pulver gemengt. Das fertige Stylingpulver wurde in Polyethylenflaschen abgefüllt.

### 2. Anwendung

Es wurden 19 Haarsträhnen mit je einem der oben genannten Stylingmittel behandelt. Dabei wurde die Haarsträhne angefeuchtet und auf die feuchte Strähne das erfindungsgemäße Mittel aufgetragen und einmassiert. Die behandelte Haarsträhne wurde auf einem Brett gestreckt fixiert und trocknen gelassen.

Für die erfindungsgemäßen Zusammensetzungen E1 bis E20 wurde ein hervorragender Halt erhalten. Das Haar besaß deutlich mehr Struktur und Textur. Trotz der eingesetzten partikelförmigen Fällungskieselsäure war keine sichtbare Mattierung des Haars zu beobachten. Das Haar behielt seinen natürlichen Glanz.

### 3. Verzeichnis der eingesetzten Rohstoffe

Die im Rahmen der Beispiele eingesetzten Rohstoffe sind wie folgt definiert:
- Amphomer^{®}: weisses Pulver; INCI-Bezeichnung: Octylacrylamide / Acrylates / Butylaminoethyl Methacrylate Copolymer (Akzo Nobel)
- Sipernat^{®} 500 LS: Fällungskieselsäure (weisses Pulver), pH-Wert (50 g/l Suspension in Wasser bei 20°C): 6,0; BET: 457 m²/g, DBP-Absorption: 325 g/100g, Na₂O-Gehalt: 0,6 %, mittlerer Teilchendurchmesser (d50): 4,5 µm, INCI-Bezeichnung: Hydrated Silica
- Sipernat^{®} 22 LS: Fällungskieselsäure (weisses Pulver), pH-Wert (50 g/l Suspension in Wasser bei 20°C): 6,2; BET: 190 m²/g, DBP-Absorption: 265 g/100g, Na₂O-Gehalt: 1,0 %, mittlerer Teilchendurchmesser (d50): 4,5 µm, INCI-Bezeichnung: Hydrated Silica
- Sipernat^{®}50: Fällungskieselsäure (weisses Pulver), pH-Wert (50 g/l Suspension in Wasser bei 20°C): 6,0; BET: 450 m²/g, DBP-Absorption: 335 g/100g, Na₂O-Gehalt: 0,6 %, mittlerer Teilchendurchmesser (d50): 40 µm, INCl-Bezeichnung: Hydrated Silica
- Sipernat^{®}2200: Fällungskieselsäure (weisses Pulver), pH-Wert (50 g/l Suspension in Wasser bei 20°C): 6,0; BET: 185 m²/g, DBP-Absorption: 245 g/100g, Na₂O-Gehalt: 1,0 %, mittlerer Teilchendurchmesser (d50): 320 µm
- Aerosil^{®} R 812 S: modifizierte, pyrogene Kieselsäure, INCI-Bezeichnung: Silica Silylate (Evonik Degussa)
- Wachsemulsion 2168/5BWCW: Wachsemulsion aus Bienenwachs und Carbaubawachs
- Kern-Hülle-Teilchen: Mischung aus 20 Gew.-% Aerosil R 812 S, 0,05 Gew.-% Amphomer, 0,02 Gew.-% Zitronensäure, 0,2 Gew.-% Natriumbenzoat und 79,73 Gew.-% Wasser. Herstellung: Wasser, Zitronensäure Monohydrat und Natriumbenzoat (sowie für A2 PVP/VA 60/40 W NP) wurden in einem Gefäß gemischt. Daraus resultierte eine Flüssigkeit mit einem pH-Wert zwischen 4,5 und 5. Diese Flüssigkeit wurde mit dem hydrophobierten Siliziumdioxidpulver Aerosil^{®} R 812 S (INCl-Bezeichnung: Silica Silylate) versetzt. Nach einer Rührzeit von jeweils 30 bis 45 Sekunden hatte sich jeweils ein stabiles Pulver gebildet. Das Amphomer (sofern enthalten) wurde als Feststoff zu dem stabilen Pulver gegeben und unter rühren untergemengt. Es ergeben sich Kern-Hülle-Teilchen im Sinne des 6. Anspruchs, die sich bei mechanischer Belastung verflüssigen.
- DOW Corning 9701 Cosmetic: Powder pulverförmig (Körnung <10 µm), Mischung aus einem vernetzten, elastomeren Silikon (INCI-Bezeichnung: Dimethicone/Vinyl Dimethicone Crosspolmyer) und Silika (INCI-Bezeichnung: Silica) (Dow Corning)
- DOW Corning^{®} 193 C fluid: Silikon-Glykol-Copolymer (INCI-Bezeichnung: PEG-12 Dimethicone) (Dow Corning)
- Cocoglycerides + 7.3 EO: Glyceride des Kokusnussfettsäureschnitts mit 7.3 Äquivalenten Ethylenoxid ethoxyliert
- Kahlwax^{®} 6240 Vegetable Wax: selbstemulgierender Wachsester (fest) (Kahl GmbH & Co.)
- Verityl^{®}AB 1000 Liquid: 1,4-Dihydroxymethylcyclohexan
- Luviskol^{®} VA 64 W: Copolymer aus Vinylpyrrolidon und Vinylacetat (60:40) (48-52% Aktivsubstanz in Wasser, INCl-Bezeichnung: VP/VA Copolymer) (BASF SE)

## Patentansprüche

1. Verwendung einer pulverförmigen Zusammensetzung, enthaltend
- mindestens eine partikelförmige, amorphe Fällungskieselsäure, mit der Maßgabe, dass in besagter Fällungskieselsäure mindestens ein kosmetischer Wirkstoff sorbiert vorliegt, der ausgewählt wird unter wachsförmigen Estern
- Kern-Hülle-Teilchen, deren Hülle Partikel mindestens eines hydrophobierten Metalloxidpulvers enthält und deren Kern eine flüssige, wässrige Phase umfasst, zur temporären Verformung keratinhaltiger Fasern.

2. Verwendung nach Anspruch 1, **dadurch gekennzeichnet, dass** die Fällungskieselsäure, bezogen auf ihr Gewicht mindestens 0,1 bis 1,0 Gew.-% Na₂O enthält.

3. Verwendung nach einem der Ansprüche 1 oder 2, **dadurch gekennzeichnet, dass** das besagte Metalloxid eine DBP-Zahl von 50 bis 800 g, bevorzugt von 100 bis 500g, besonders bevorzugt von 200 bis 400 g, ganz besonders bevorzugt von 250 bis 350 g, jeweils pro 100 g Metalloxid (gemessen nach ASTM D 2414) besitzt.

4. Verwendung nach einem der Ansprüche 1 oder 2, **dadurch gekennzeichnet, dass** das besagte Metalloxid in einer 5 Gew.-%-igen Aufschlämmung in destilliertem Wasser einen pH-Wert der resultierenden Mischung von 4,5 bis 8,0, insbesondere von 5,0 bis 7,5, bewirkt.

5. Verwendung nach einem der Ansprüche 1 bis 3, **dadurch gekennzeichnet, dass** das besagte Metalloxid eine BET-Oberfläche von 50 bis 1000 m²/g, bevorzugt von 100 bis 750 m²/g, besonders bevorzugt von 150 bis 500 m²/g (jeweils gemessen nach ISO 5794-1), besitzt.

6. Verwendung nach Anspruch 1, **dadurch gekennzeichnet, dass** das hydrophobierte Metalloxid des hydrophobierten Metalloxidpulvers ausgewählt wird aus mindestens einem Vertreter der Gruppe, die gebildet wird aus hydrophobierten Silikaten, hydrophobierten Aluminiumsilikaten, hydrophobiertem Titandioxid sowie hydrophobiertem Siliziumdioxid.

7. Verwendung nach einem der Ansprüche 1 oder 6, **dadurch gekennzeichnet, dass** das hydrophobierte Metalloxidpulver ausgewählt wird aus Silica Silylate.

8. Verwendung nach einem der Ansprüche 6 bis 7, **dadurch gekennzeichnet, dass** die Kern-Hülle-Teilchen in der flüssigen, wässrigen Phase bezogen auf das Gewicht der Kern-Hülle-Teilchen der pulverförmigen Zusammensetzung 70 Gew.-% bis 90 Gew.-% eines wässrigen Lösungsmittels enthalten.

9. Verwendung nach einem der Ansprüche 1 und 6 bis 8, **dadurch gekennzeichnet, dass** die Kern-Hülle-Teilchen geeignet sind, die flüssige, wässrige Phase des Kerns durch mechanische Belastung der Kern-Hülle-Teilchen (insbesondere durch Reibung und/oder Druck) aus dem Kern-Hülle-Teilchen freizusetzen und dabei aus der pulverförmigen Zusammensetzung eine Flüssigkeit zu bilden.

10. Verwendung nach einem der Ansprüche 1 bis 9, **dadurch gekennzeichnet, dass** besagter kosmetischer Wirkstoff (bzw. Mischungen davon) in einem Gewichtsverhältnisbereich von 1 zu 1 bis 9 zu 1, insbesondere von 3 zu 1 bis 4 zu 1, im partikelförmigen, amorphen Metalloxid sorbiert vorliegt.

11. Verfahren zur kosmetischen Behandlung keratinhaltiger Fasern, insbesondere menschlicher Haare, in dem
a) die keratinhaltigen Fasern gegebenenfalls angefeuchtet werden,
b) eine pulverförmige Zusammensetzung enthaltend
- mindestens eine partikelförmige, amorphe Fällungskieselsäure, mit der Maßgabe, dass in besagter Fällungskieselsäure mindestens ein kosmetischer Wirkstoff sorbiert vorliegt, der ausgewählt wird unter wachsförmigen Estern,
- Kern-Hülle-Teilchen, deren Hülle Partikel mindestens eines hydrophobierten Metalloxidpulvers enthält und deren Kern eine flüssige, wässrige Phase umfasst
auf die keratinhaltigen Fasern aufgetragen wird,
c) diese pulverförmige Zusammensetzung während oder nach dem Auftragen auf die keratinhaltigen Fasern einer mechanischen Belastung ausgesetzt wird, wobei sich die pulverförmige Zusammensetzung in eine Flüssigkeit umwandelt,
d) die aufgetragene Zusammensetzung auf die keratinhaltigen Fasern einwirkt und gegebenenfalls ausgespült wird.

## Claims

1. Use of a powdery composition, containing
- at least one particulate amorphous precipitated silicic acid, with the condition that at least one cosmetic active ingredient, selected from waxy esters, is present in an absorbed state in the precipitated silicic acid,
- core-shell particles, the shells of which contain particles of at least one hydrophobized metal oxide powder and the cores of which comprise a liquid, aqueous phase,
for temporarily deforming keratin-containing fibers.

2. The use according to claim 1, **characterized in that** the precipitated silicic acid contains at least from 0.1 to 1.0 wt.% Na₂O, based on its weight.

3. The use according to either claim 1 or claim 2, **characterized in that** the metal oxide has a DBP number of from 50 to 800 g, preferably from 100 to 500 g, more preferably from 200 to 400 g, most preferably from 250 to 350 g, in each case per 100 g metal oxide (measured according to ASTM D 2414).

4. The use according to either claim 1 or claim 2, **characterized in that** in a 5 wt.% suspension in distilled water, the metal oxide causes the resulting mixture to have a pH of from 4.5 to 8.0, in particular of from 5.0 to 7.5.

5. The use according to one of claims 1 to 3, **characterized in that** the metal oxide has a BET surface area of from 50 to 1000 m²/g, preferably from 100 to 750 m²/g, more preferably from 150 to 500 m²/g (measured in each case according to ISO 5794-1).

6. The use according to claim 1, **characterized in that** the hydrophobized metal oxide of the hydrophobized metal oxide powder is selected from at least one representative of the group which is formed by hydrophobized silicates, hydrophobized aluminum silicates, hydrophobized titanium dioxide, and hydrophobized silicon dioxide.

7. The use according to either claim 1 or claim 6, **characterized in that** the hydrophobized metal oxide powder is selected from silica silylates.

8. The use according to one of claims 6 or 7, **characterized in that**, in the liquid aqueous phase, the core-shell particles contain from 70 wt.% to 90 wt.% of an aqueous solvent, based on the weight of the core-shell particles of the powdery composition.

9. The use according to one of claims 1 and 6 to 8, **characterized in that** the core-shell particles are suitable for releasing the liquid aqueous phase of the core from the core-shell particles by means of mechanical loading of the core-shell particles (in particular by friction and/or pressure) and for forming a liquid from the powdery composition.

10. The use according to one of claims 1 to 9, **characterized in that** the cosmetic active ingredient (or mixtures thereof) is present in an absorbed form in particulate amorphous metal oxide in a weight-ratio range of from 1:1 to 9:1, in particular from 3:1 to 4:1.

11. A method for cosmetic treatment of keratin-containing fibers, in particular human hair, in which
a) the keratin-containing fibers are optionally moistened,
b) a powdery composition containing
- at least one particulate amorphous precipitated silicic acid, with the condition that at least one cosmetic active ingredient, selected from waxy esters, is present in an absorbed form in the precipitated silicic acid,
- core-shell particles, the shells of which contain particles of at least one hydrophobized metal oxide powder and the core of which comprises a liquid aqueous phase,
is applied to the keratin-containing fibers,
c) this powdery composition being exposed to a mechanical load either during or after application to the keratin-containing fibers, wherein the powdery composition transforms into a liquid,
d) the applied composition acts upon the keratin-containing fibers, and is optionally rinsed out.

## Revendications

1. Utilisation d'une composition en poudre contenant
- au moins un précipité d'acide silicique amorphe particulaire, avec la condition que ledit précipité d'acide silicique contienne au moins un ingrédient actif cosmétique sous forme sorbée qui est choisi parmi les esters cireux,
- des particules noyau-enveloppe dont l'enveloppe contient des particules d'au moins une poudre d'oxyde métallique hvdrophobisé et dont le noyau comprend une phase aqueuse liquide,
pour la mise en forme temporaire de fibres de kératine.

2. Utilisation selon la revendication 1, **caractérisée en ce que** le précipité d'acide silicique contient, sur la base de son poids, au moins 0,1 à 1,0% de Na₂O.

3. Utilisation selon l'une des revendications 1 ou 2, **caractérisée en ce que** ledit oxyde métallique a un indice DBP de 50 à 800 g, de préférence de 100 à 500 g, de manière particulièrement préférée de 200 à 400 g, de manière tout particulièrement préférée de 250 à 350 g, pour 100 g d'oxyde métallique (mesuré selon la norme ASTM D 2414).

4. Utilisation selon l'une des revendications 1 ou 2, **caractérisée en ce que** ledit oxyde métallique, dans une suspension de 5% en poids dans de l'eau distillée, entraîne une valeur de pH du mélange résultant de 4,5 à 8,0, en particulier 5,0 à 7,5.

5. Utilisation selon l'une des revendications 1 à 3, **caractérisée en ce que** ledit oxyde métallique a une surface BET de 50 à 1000 m²/g, de préférence de 100 à 750 m²/g, de manière particulièrement préférée de 150 à 500 m²/g (mesurée à chaque fois selon la norme ISO 5794-1).

6. Utilisation selon la revendication 1, **caractérisée en ce que** l'oxyde métallique hydrophobisé de la poudre d'oxyde métallique hvdrophobisé est choisi parmi au moins un représentant du groupe qui est formé par les silicates hvdrophobisés, les silicates d'aluminium hydrophobisés, le dioxyde de titane hydrophobisé et le dioxyde de silicium hydrophobisé.

7. Utilisation selon l'une des revendications 1 ou 6, **caractérisée en ce que** l'oxyde métallique hydrophobisé est choisi parmi le silylate de silice.

8. Utilisation selon l'une des revendications 6 ou 7, **caractérisée en ce que** les particules noyau-enveloppe dans la phase aqueuse liquide contiennent, par rapport au poids des particules noyau-enveloppe de la composition en poudre, de 70% en poids à 90% en poids d'un solvant aqueux.

9. Utilisation selon l'une des revendications 1 ou 6 à 8, **caractérisée en ce que** les particules noyau-enveloppe sont appropriés pour que la phase aqueuse liquide du noyau soit libérée des particules à noyau-enveloppe par contrainte mécanique des particules noyau-enveloppe (en particulier par frottement et/ou par pression) et pour qu'elle forme alors un liquide à partir de la composition en poudre.

10. Utilisation selon l'une des revendications 1 à 9, **caractérisée en ce que** ladite substance active cosmétique (ou des mélanges de celle-ci) est présente sous forme sorbée dans l'oxyde amorphe métal dans une gamme de rapports en poids de 1:1 à 9:1, en particulier de 3:1 à 4:1.

11. Procédé de traitement cosmétique de fibres de kératine, et en particulier de cheveux humains, dans lequel
a) les fibres de kératine sont éventuellement humidifiées,
b) une composition en poudre contenant
- au moins une précipité d'acide silicique amorphe particulaire, à la condition que dans ledit précipité d'acide silicique au moins une substance active cosmétique est se présente sous forme sorbée qui est choisie parmi les esters cireux,
- de particules noyau-enveloppe dont l'enveloppe contient des particules d'au moins une poudre d'oxyde métallique hvdrophobisée et dont le noyau comprend une phase aqueuse liquide,
est appliquée sur les fibres de kératine,
c) cette composition en poudre est soumise pendant ou après l'application sur les fibres de kératine à une charge mécanique, la composition en poudre étant transformée en un liquide,
d) la composition appliquée agit sur les fibres de kératine, et éventuellement rincée.
